# EUROPEAN PATENT APPLICATION

(11) **EP 4 571 386 A1**
(43) Date of publication of application: **18.06.2025**
(21) Application number: 23216069.7
(22) Date of filing: 12.12.2023
(51) Int. Cl.: G02B 21/00, A61B 3/13, A61B 3/14, G02B 21/36, A61B 3/00

(54) **MEDICAL IMAGING SYSTEM**

(71) Applicant: Leica Instruments (Singapore) Pte Ltd, Singapore 608924 (SG)
(72) Inventor: Zeng, Zhengdong, 608924 Singapore (SG); Schutz, Marco, 608924 Singapore (SG); Leck, Kheng Swee, 608924 Singapore (SG); Kok, Alvin, 608924 Singapore (SG); Yang, Gao, 608924 Singapore (SG)
(74) Representative: 2SPL Patentanwälte PartG mbB

(57) **Abstract**

The invention basically refers to a medical imaging system (200), preferably a surgical imaging system, comprising an illumination system (220) and an imaging sensor (230), wherein the illumination system (220) comprises a flat lens (222) and at least one illumination light source (210), wherein the flat lens (222) and the at least one illumination light source (210) are aligned together with the imaging sensor (130, 230) along an optical path (216) of light originating from the at least one illumination light source, wherein the flat lens (222) is located, along the optical path (216), between the imaging sensor (230) and the at least one illumination light source (210).

## Description

### Technical Field

The present invention essentially relates to a medical imaging system comprising an illumination system and an imaging sensor, wherein the illumination system comprises a flat lens and at least one illumination light source.

### Background

In medical or surgical microscopy, e.g., in ophthalmology or other applications, imaging systems can be used by a surgeon to view a desired object, e.g., a patient's eye, including proper illumination.

### Summary

In view of the situation described above, there is a need for improvement in providing a view and illumination of a desired object in medical applications. According to an embodiment of the invention, a medical imaging system with the features of the independent claim is proposed. Advantageous further developments form the subject matter of the dependent claims and of the subsequent description.

An embodiment of the invention relates to a medical (or preferably surgical) imaging system. The medical imaging system comprises an illumination system and an imaging sensor. The illumination system comprises a flat lens and at least one illumination light source, wherein the flat lens and the at least one illumination light source (210) are aligned together with the imaging sensor along an optical path of light originating from the at least one illumination light source. The flat lens is located, along the optical path, between the imaging sensor and the at least one illumination light source. For example, the flat lens can have an object side and an illumination side, wherein the at least one illumination light source is arranged at the illumination side of the flat lens.

The inventors have recognized that using flat lenses or can address the pain points of traditional medical imaging systems, e.g., microscope optics carriers thereof. Traditional lenses use curved surfaces and multiple components that make the imaging system large, heavy, and difficult to maneuver. In contrast, flat lenses use a flat surface to manipulate light waves and are thinner, more compact, and more durable than traditional lenses. For example, a thickness of the flat lens, along the optical path, is less than 200 µm or less than 50 µm or less than 20 µm. The flat lenses can also bend light in different directions, manipulate the polarization of light, and focus light without the need for curved surfaces, providing new and innovative applications in medical imaging.

Integrating flat lens technology into the medical microscope optics carrier, for example, can improve accuracy, portability, durability, and cost-effectiveness. Medical professionals can benefit from improved image acquisition and analysis, more accessible and useful microscopes, and cost savings due to simpler manufacturing processes. The benefits of this technology make it an ideal choice for the illumination module of the medical imaging system or a medical microscope optics carrier, allowing for a reduction in size and weight, increased durability, and the ability to manipulate light waves in new and unique ways.

In an embodiment, the imaging system comprises an objective lens, wherein the objective lens is arranged at the object side of the flat lens. This allows for a compact imaging system due to the flat lens.

In an embodiment, the flat lens is, optically, arranged next to the at least one illumination light source. In other words, there is no optically effective element between the flat lens and light source. This allows a very compact imaging system. In an embodiment, the flat lens is, optically, arranged next to the objective lens. In other words, there is no optically effective element (e.g., lenses or apertures) between the flat lens and the objective lens; a mirror and/or a beam splitter, however, can be arranged between the flat lens and the objective lens.

In an embodiment, the flat lens comprises multiple structures, e.g., microstructures. These structures are distributed along at least one of the two outer surfaces of the flat lens (i.e., front or back surface), which outer surfaces are oriented orthogonal to the optical axis. The structures influence the propagation of the light originating from the at least one illumination light source. Each structure or microstructure can have a unique design, for example, consisting of numerous microelements. In this way, the flat lens and, thus, the medical imaging system can be tailored to specific needs with a very compact size.

In an embodiment, at least one of the multiple structures is configured to collect incident illumination light from the illumination side and provide a homogeneous illumination in an object plane at the object side. This allows improved illumination by the medical imaging system.

In an embodiment, the at least one illumination light source comprises an infrared light source or a near-infrared light source. In this case, geometric dimensions of the multiple structures can be configured for infrared or near-infrared light. This allows for compact infrared medical imaging.

In an embodiment, the at least one illumination light source comprises one or more LEDs (Light Emitting Diodes). In an embodiment, the at least one illumination light source comprises one or more VCSELs (Vertical-cavity surface-emitting laser). A combination of LEDs and VCELs is also possible. The LEDs and/or VCSELs can have different wavelength of visible light, e.g., blue, red, green light, white light or a combination thereof This allows for compact visible light medical imaging.

In an embodiment, the medical imaging system is configured as ophthalmologic medical imaging system. This allows compact ophthalmology due to the flat lens technology.

In an embodiment, the medical imaging comprises a movable mount unit, wherein the movable mount unit comprises, preferably fully enclosed in a housing of the movable mount unit, the illumination system with the flat lens and the objective lens. This allows a very compact movable mount unit due to the flat lens technology.

In an embodiment, the medical imaging system comprises a mirror, wherein the mirror is located along and within the optical path. This allows diverting the geometric orientation of the light paths, resulting in an even more compact medical imaging system.

In an embodiment, the flat lens is directly arranged on or integrated with one of the at least one illumination light source, e.g., the VCSEL mentioned above or another laser diode. This allows for a very compact imaging system, similar to a lidar sensor (with a refractive lens) arranged on a VCSEL in, e.g., smartphones, with the difference that a flat lens is used instead of such lidar sensor.

In an embodiment, the medical imaging system further comprises at least one of: an eyepiece, a display. The medical imaging system is configured to display an image acquired by means of the imaging sensor in or on the at least one of the eyepiece and the display. This allows for a compact imaging system for, e.g., surgeons and/or people watching a surgery.

In an embodiment, the imaging flat lens is configured for retina imaging, i.e., the imaging flat lens is configured as objective lens of the imaging system for a retina of an eye (where the retina or the eye is the object to be imaged).

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

### Short description of the Figures

- Fig. 1: schematically shows a medical imaging system according to an embodiment of the invention;
- Fig. 2a: schematically shows a medical imaging system according to another embodiment of the invention;
- Fig. 2b: schematically shows a part of the medical imaging system of Fig. 2a in another view;
- Fig. 3a: schematically shows a part of the medical imaging system of Fig. 2a in another view; and
- Fig. 3b: schematically a conventional medical imaging system for comparison.

### Detailed Description

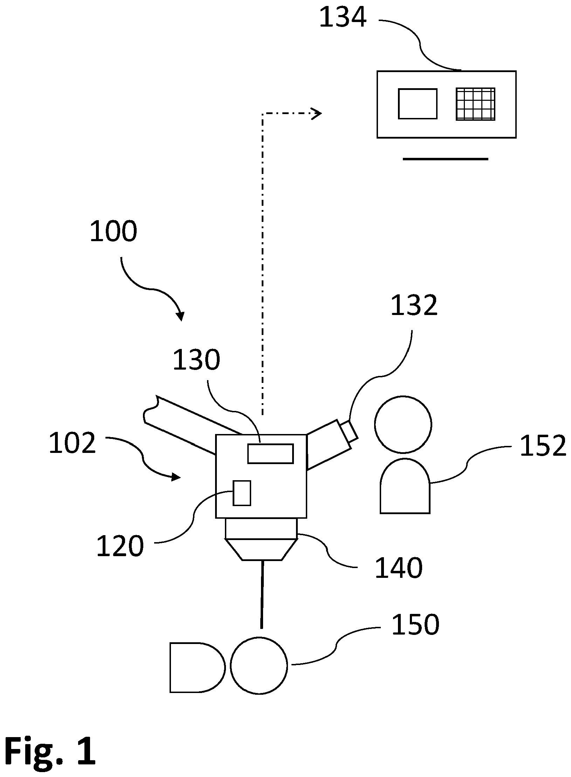

Fig. 1 schematically illustrates a medical (or surgical) imaging system 100 according to an embodiment of the invention. For example, the medical imaging system 100 can be configured as a surgical microscope. The medical imaging system 100 comprises an illumination system 120 and an imaging sensor 130. The medical imaging system 100 further comprises, by means of example, an objective lens 140.

In an embodiment, the medical imaging system 100 comprises a movable mount unit 102, wherein the movable mount unit 102 comprises, preferably fully enclosed in a housing of the movable mount unit, the illumination system 120 and the objective lens 140. In addition, the movable mount unit 102 can comprise, preferably fully enclosed in the housing of the movable mount unit, the imaging sensor 130.

In an embodiment, the medical imaging system 100 comprises an eyepiece 132 and a display 134, wherein the medical imaging system 100 is configured to display an image acquired by means of the imaging sensor 130 in the eyepiece and/or on the display. It is noted that only the eyepiece 132 or only the display 134 can be provided, for example. Using both, however, improves the possibilities for viewing the object. For example, a surgeon 152 can use the medical imaging system viewing or imaging an object 150, e.g., an eye of a patient.

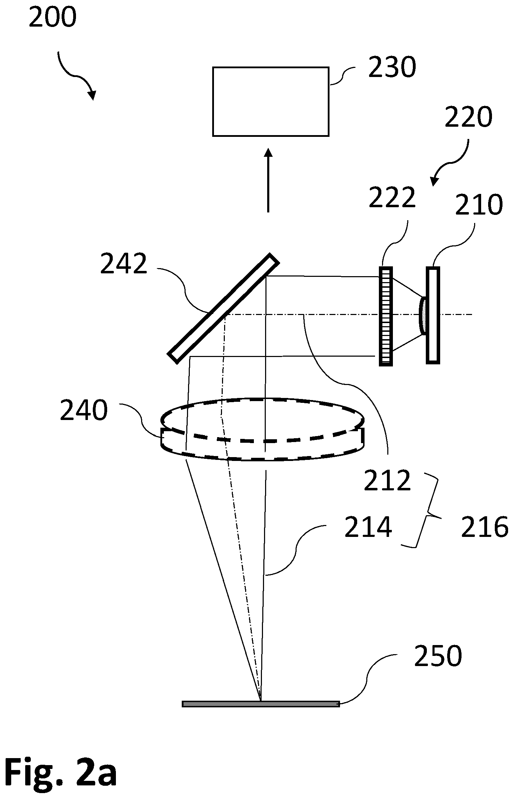

Fig. 2a schematically illustrates a medical (or surgical) imaging system 200 according to another embodiment of the invention. Basically, the medical imaging system 200 can correspond to or be similar to the medical imaging system 100 according to Fig. 1, however, different details are shown in Fig. 2a compared to Fig. 1. Like element or components are referred to with like reference numerals (with a leading 2 instead of leading 1).

The medical imaging system 200 comprises an illumination system 220 and an imaging sensor 230. The medical imaging system 200 further comprises, by means of example, an objective lens 240 and a mirror 242. The medical imaging system 200 can be used to illuminate and view an object 250. In the schematic view of Fig. 2a, the object 250 can also be seen as an object plane.

The illumination system 220 comprises a flat lens 222 and an illumination light source 210, wherein the flat lens 222 and the illumination light source 210 are aligned together with the imaging sensor 230 along an optical path 216 of light originating from the illumination light source 210. The optical path 216 can comprise an illumination path 212 from the illumination source 210 to the object, and an imaging path 214, from the object 250 to the imaging sensor 230. It is noted that the illumination path 212 and the imaging path 214 can overlap.

In an embodiment, the illumination light source 210 comprises an infrared or a near-infrared light source, i.e., a light source having a wavelength of, e.g, between 780 nm and 1000 nm. Also, higher upper limits, e.g. 1.400 nm or even 3.000 nm can be used, depending on need. In an embodiment, the illumination light source 210 comprises one or more LEDs, laser diodes or VCSELs or a combination thereof. This allows choosing the wavelength according to needs. For example, such illumination light source can provide white light (or visible light), blue, red, green or other colors or arbitrary combinations thereof. Also, individual use of individual LEDs might be possible, providing, e.g., white, blue or red light at different times. Further, two illumination light sources could be provided, e.g., a infrared one and a white light one.

The flat lens 222 is located, along the optical path 216, between the imaging sensor 230 and the illumination light source 210. Further, the mirror 242 is also located along and within the optical path 216. It is noted that the mirror 242 can be, for example, a half mirror (partly reflecting light, partly transmitting light) or a full mirror (fully reflecting light) that is only partly inserted into the optical path (which has a certain width). For example, the mirror 242 can also be a scanning mirror; the mirror 242 can also be a fixed mirror.

In embodiment, the flat lens 222 is, optically, arranged next to the illumination light source 210, i.e., there is no separate, optically effective element like a conventional lens in-between. In an embodiment, the flat lens 222 can also be directly arranged on the illumination light source 210 or even be integrated with the illumination light source 210. This may, for example, require appropriate means for fixing the flat lens 222 onto the illumination light source 210.

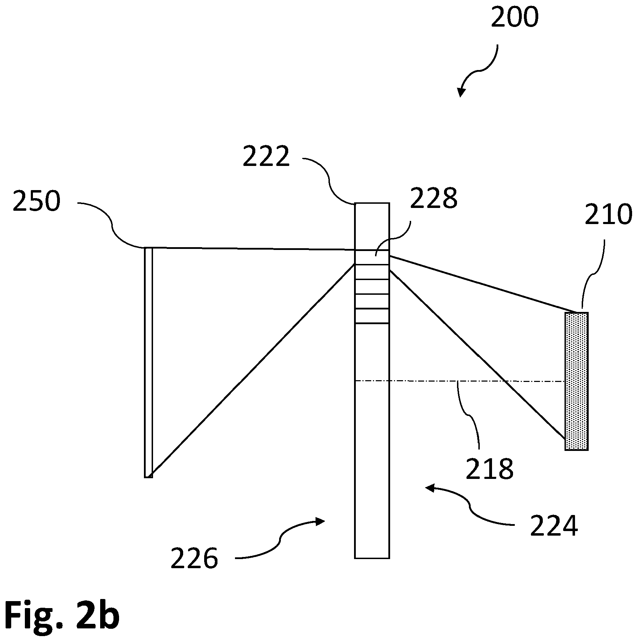

Fig. 2b illustrates parts of the medical imaging system 200 of Fig. 2b in another view to explain the flat lens 222. For illustration purposes, only the illumination source 210, the flat lens 222 and the object or object plane 250 are shown (due to the missing mirror, the optical path is not diverted in this case, however, this does not affect the explanation). The flat lens 222 has an object side 226 and an illumination side 224, wherein the illumination light source 210 is arranged at the illumination side 224 of the flat lens 222. The objective lens 240 (see Fig. 2a) is arranged at the object side 226 of the flat lens 222, i.e., in the optical path 216 between the flat lens 222 and the object 250.

Further, Fig. 2b illustrates that the flat lens 222 comprises multiple structures 228 (e.g., microstructures), that are distributed along at least one of the two outer surfaces of the flat lens, which outer surfaces are oriented orthogonal to the optical axis 218. The optical axis 218 can be aligned with the optical path 216 or the illumination path 212, at least at the location of the flat lens 222, for example. The two outer surfaces are, thus, oriented at the illumination side 224 and the object side 226.

The structures 228 influence the propagation of the light originating from the illumination light source 210. In particular, at least one of the multiple structures 228 is configured to collect incident illumination light from the illumination side 224 and provide a homogeneous illumination in the object plane 250 (or at the object) at the object side 226. This is schematically illustrated in Fig. 2b. Geometric dimensions of the multiple structures can then be configured for infrared or near-infrared light (if such illumination light source is used) or to other wavelengths. In general, the size and/or arrangement of these structures or microstructures can be tailored to the desired propagation of light.

It is noted that the objective lens 240 to be located between the flat lens 222 and the object or object plane 250 can or should also be considered when designing the flat lens 222 or its structures 228 such that a homogeneous illumination in the object plane 250 is provided.

A flat lens, in general, can be a lens whose flat shape allows it to provide distortion-free-imaging, potentially with arbitrarily-large apertures. Flat lenses can require a refractive index close to -1 over a broad angular range, however flat lenses based on metasurface can also be used. A metasurface or electromagnetic metasurface refers to a kind of artificial sheet material with sub-wavelength thickness. Metasurfaces can be either structured or unstructured with subwavelength-scaled patterns in the horizontal dimensions. A metasurface can be an electromagnetic structure engineered on subwavelength scales to elicit tailored polarization responses. Metamaterials can, for example, image infrared, visible, and also ultraviolet wavelengths.

In contrast to flat lenses, traditional curved glass lenses can bend light coming from many angles to end up at the same focal point on a piece of photographic film or an electronic sensor. Light captured at the very edges of a curved glass lens does not line up correctly with the rest of the light, creating a fuzzy image at the edge of the frame. To correct this, lenses use extra pieces of glass, adding bulk, complexity, and mass.

Thus, with the proposed use of flat lenses for imaging and illumination, a very thin illumination system can be provided. This will be illustrated by comparing the flat lens system to a conventional system in the following.

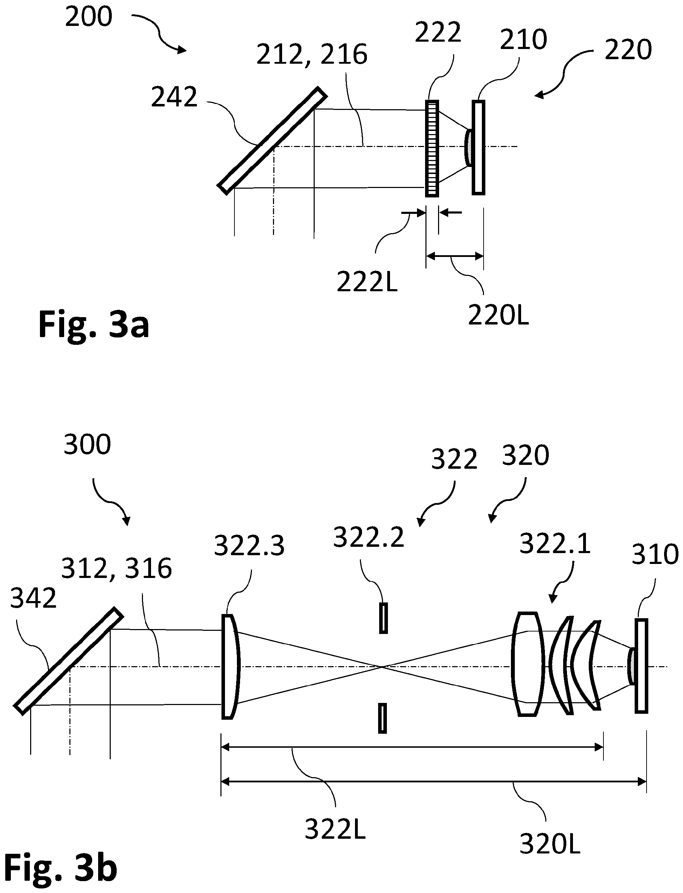

Fig. 3a illustrates the medical imaging system 200 and, in particular, the illumination system 220 comprising the illumination light source 210 and the flat lens 222. In addition, the mirror 242 is shown. Further, the illumination path 212 and the optical path 216 (in this case both are the same) are shown.

A length or thickness of the flat lens 222, along the optical path 216, is referred to by reference numeral 222L, and a length of the illumination system 220, comprising the illumination light source 210 and the flat lens 222, along the optical path 216, is referred to by reference numeral 220L. The thickness 222L (note that in case of the flat lens, it is more a thickness rather than a length) of the flat lens 222 is, in an embodiment, less than 200 µm or less than 50 µm or less than 20 µm. This, in particular, refers to the flat lens as such. It is noted that the flat lens can, for example, also be adhered to a substrate to improve strength and/or stability. The overall thickness including the flat lens as such and the substrate can the be more than 200 µm, even though the thickness of the flat lens as such is less than 200 µm for example; this is since the entire thickness depends largely on the thickness of the substrate which can vary largely. Irrespective of that, also the flat lens as such can have a larger thickness, if so desired.

The size of the illumination system 220 is illustrated in Fig. 2a by the length L, which is the overall length of the flat lens 222 and the illumination light source 210 along the optical path 216.

Fig. 3b illustrates medical imaging system 300 and, in particular, an illumination system 320. The illumination system 300, as to basic functions, can correspond to illumination system 220; a main difference, however, is that a conventional optical system 322 comprising multiple lenses, optical elements and the like is used. In addition, a mirror 342 is shown. Further, an illumination path 312 and an optical path 316 (in this case both are the same) are shown.

In particular, the illumination system 300 comprises an illumination light source 310 and the optical system 322, which comprises a collection lens system 322.1 (e.g., three lenses), an aperture 322.2, and a condenser lens 322.3.

A length of the optical system 322, along the optical path 316, is referred to by reference numeral 322L, and a length of the illumination system 320, comprising the illumination light source 310 and the optical system 322, along the optical path 316, is referred to by reference numeral 320L. Due to the use of conventional lenses, the light-bending power is limited, and the lenses are thicker and heavier than a flat lens.

As can be seen in comparison with Fig. 3a, the length 322L of the optical system 322 much bigger than the length (or thickness) 222L of the flat lens 222. Although the distance between the flat lens 222 and the illumination system 210 (i.e., the difference between 220L and 222L) can be similar to the distance between the optical system 322 and the illumination system 310 (i.e., the difference between 320L and 322L), the flat lens results also in a much shorter illumination system 200, compared to the illumination system 300. This allows a much more compact and smaller medical imaging system.

The proposed integration of flat lens technology into the medical imaging system or microscope optics carrier addresses the pain points of the large size and weight of traditional medical imaging system microscope optics carriers as can be seen in Figs. 3a and 3b. The illumination system or module of traditional microscopes typically uses multiple traditional lenses, which makes the microscope large, heavy, and difficult to maneuver. By using flat lenses in the illumination module, the microscope can be made thinner, more compact, and lighter, making it more portable and easier to use in various settings. This addresses the customer pain point of the ergonomic challenges associated with the large size and weight of traditional microscope optics carriers.

The current design of a traditional microscope optics carrier has several pain points that can be addressed by the integration of flat lens optics technology. One of the primary pain points is the large size of the illumination system or module, which is typically composed of multiple traditional optics lenses. This results in a huge and heavy optics carrier, leads to ergonomic challenges from the stack height and difficulties in maneuvering the system.

Flat lens technology is a type of lens technology that uses a flat surface to manipulate light waves, as opposed to traditional lenses that use curved surfaces. This technology has several benefits over traditional lens designs, including a reduction in size and weight, increased durability, and the ability to manipulate light waves in new and unique ways.

The flat lenses used in the illumination system or module of the medical microscope optics carrier allow for a reduction in size and weight, as they are thinner and more compact than traditional lenses. This reduction in size and weight makes the microscope more portable and easier to use in various settings, which is particularly important for medical professionals working in remote or underserved areas.

In addition to their size and weight benefits, flat lenses are also more durable than traditional lenses. Traditional lenses are often made from glass or plastic, which can be fragile and prone to cracking or breaking. Flat lenses, on the other hand, are typically made from materials such as silicon or gold, which are more robust and less likely to break or degrade over time.

Integrating NIR (near-infrared) technology with flat lens technology in the illumination system can address pain points in medical imaging or microscopy and provide several benefits for medical professionals. NIR light can penetrate deeper into tissue, providing clearer images of structures that are deeper within the body in low light conditions.

The unique way in which flat lenses manipulate light waves allows for new and innovative applications in optics and, in particular, in medical optics and imaging. Flat lenses can be designed to bend light in different directions, manipulate the polarization of light, and even focus light without the need for curved surfaces. These capabilities have the potential to revolutionize medical microscopy, by allowing for more accurate and precise image acquisition and analysis.

In conclusion, flat lens technology is a promising new development in the field of medical optics and imaging. The benefits of this technology, including a reduction in size and weight, increased durability, and the ability to manipulate light waves in new and unique ways, make it an ideal choice for the illumination module of the medical microscope optics carrier. By integrating flat lens technology into the microscope, medical professionals can benefit from improved accuracy, portability, and ease of use. The medical imaging system described herein, in its various embodiments, can be used in various medical applications like surgeries.

As used herein the term "and/or" includes any and all combinations of one or more of the associated listed items and may be abbreviated as "f".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 200, 300: medical imaging system
- 102: movable mount unit
- 110, 210, 310: illumination light source
- 120, 220, 320: illumination system
- 130, 230: imaging sensor
- 132: eyepiece
- 134: display
- 140, 240: objective lens
- 150, 250: object
- 152: surgeon

- 212: illumination path
- 214: imaging path
- 216: optical path
- 218: optical axis
- 220L, 230L: illumination system length
- 222: flat lens
- 222L: flat lens width
- 224: illumination side
- 226: object side
- 228: structures
- 242, 342: mirror

- 322: optical system
- 322L: optical system length
- 322.1: collection lens system
- 322.2: aperture
- 322.3: condenser lens

## Claims

1. A medical imaging system (100, 200), preferably a surgical imaging system, comprising an illumination system (120, 220) and an imaging sensor (130, 230),
wherein the illumination system (120, 220) comprises a flat lens (222) and at least one illumination light source (210), wherein the flat lens (222) and the at least one illumination light source (210) are aligned together with the imaging sensor (130, 230) along an optical path (216) of light originating from the at least one illumination light source,
wherein the flat lens (222) is located, along the optical path (216), between the imaging sensor (130, 230) and the at least one illumination light source (110, 210).

2. The medical imaging system (100, 200) of claim 1, wherein the flat lens has an object side (226) and an illumination side (224), and wherein the at least one illumination light source (110, 210) is arranged at the illumination side (224) of the flat lens (222).

3. The medical imaging system (100, 200) of claim 2, further comprising an objective lens (140, 240), wherein the objective lens is arranged at the object side (226) of the flat lens (222).

4. The medical imaging system (100, 200) of any one of the preceding claims, wherein the flat lens (222) is, optically, arranged next to the at least one illumination light source (110, 220).

5. The medical imaging system (100, 200) of any one of the preceding claims, wherein the flat lens (222) comprises multiple structures (228), that are distributed along at least one of the two outer surfaces of the flat lens, which outer surfaces are oriented orthogonal to an optical axis (218), wherein the structures (228) influence the propagation of the light originating from the at least one illumination light source (110, 210).

6. The medical imaging system (100, 200) of claim 5, wherein at least one of the multiple structures (228) is configured to collect incident illumination light from the illumination side and provide a homogeneous illumination in an object plane (250) at the object side.

7. The medical imaging system (100, 200) of any one of the preceding claims, wherein the at least one illumination light source (110, 210) comprises an infrared light source or a near-infrared light source.

8. The medical imaging system (100, 200) of claim 5 or 6 and of claim 7, wherein geometric dimensions of the multiple structures (228) are configured for infrared or near-infrared light.

9. The medical imaging system (100, 200) of any one of the preceding claims, wherein the at least one illumination light source (110, 210) comprises one or more LEDs or one or more laser diodes or VCSELs.

10. The medical imaging system (100, 200) any one of the preceding claims, configured as at least one of: an ophthalmologic medical imaging system, a microscope.

11. The medical imaging system (100, 200) of any one of the preceding claims, referring back to claim 3, comprising a movable mount unit (102), wherein the movable mount unit comprises, preferably fully enclosed in a housing of the movable mount unit, the illumination system with the flat lens and the objective lens.

12. The medical imaging system (100, 200) of any one of the preceding claims, comprising a mirror (242), wherein the mirror is located along and within the optical path (216).

13. The medical imaging system (100, 200) of any one of the preceding claims, wherein a thickness (222L) of the flat lens, along the optical path (216), is less than 200 µm or less than 50 µm or less than 20 µm.

14. The medical imaging system (100, 200) of any one of the preceding claims, wherein the flat lens is directly arranged on or integrated with one of the at least one illumination light source.

15. The medical imaging system (100, 200) of any one of the preceding claims, further comprising at least one of: an eyepiece (132), a display (134), wherein the medical imaging system is configured to display an image acquired by means of the imaging sensor (130, 230) in or on the at least one of the eyepiece (132) and the display (134).
